# EUROPEAN PATENT APPLICATION

(11) **EP 3 275 467 A1**
(43) Date of publication of application: **31.01.2018**
(21) Application number: 16305961.1
(22) Date of filing: 26.07.2016
(51) Int. Cl.: A61K 48/00, C12N 15/864

(54) **PHARMACEUTICAL COMPOSITIONS FOR THE TREATMENT OF LEFT VENTRICULAR HYPERTROPHY AND DYSFUNCTION ASSOCIATED TO METABOLIC SYNDROME**

(71) Applicant: INSERM (Institut National de la Santé et de la Recherche Médicale), 75013 Paris (FR); Université Paul Sabatier Toulouse III, 31400 Toulouse (FR)
(72) Inventor: ROUET, Philippe, 31432 Toulouse cedex 4 (FR); SMIH-ROUET, Fatima, 31432 Toulouse cedex 4 (FR)
(74) Representative: Cabinet Plasseraud

(57) **Abstract**

The present invention relates to methods and pharmaceutical compositions for the treatment of left ventricular hypertrophy and dysfunction associated to metabolic syndrome. In particular, the present invention relates to a method of treating left ventricular hypertrophy and dysfunction associated to metabolic syndrome in a subject in need thereof comprising administering to the subject a therapeutically effective amount of at least one agent capable of raising the expression of IGFBP2.

## Description

### FIELD OF THE INVENTION:

The present invention relates to methods and pharmaceutical compositions for the treatment of left ventricular hypertrophy and dysfunction associated to metabolic syndrome.

### BACKGROUND OF THE INVENTION:

Metabolic Syndrome (MetS) is a disorder of energy balance that affects a quarter of the world's population and represents a leading health concern due to its link to cardiovascular disease (CVD)^{1,2,3}. The International Diabetes Federation (IDF)⁴ for Europeans defines MetS as a central obesity (waist circumference ≥ 94cm in men and ≥80cm in women) plus any two of the following criteria: elevated plasma triglyceride (TG) levels (≥150 mg/dl) or a specific treatment for lipid abnormalities, reduced high-density lipoproteins (HDL) (<40 mg/dl for men and 50 mg/dl for women), increased blood pressure (≥130 mmHg systolic or ≥85 mmHg diastolic) or treatment for hypertension, increased fasting plasma glucose (≥100 mg/dl) or treatment with hypoglycemic agent. The IDF criteria are similar to criteria established by World Health Organization (WHO)⁵ and American National Cholesterol Education Program (NCEP)⁶, but focuses on abdominal obesity as a principal criterion and adopts ethnicity-specific cut-points for elevated waist circumference. This results in a higher prevalence of MetS⁷, and identifies more individuals at excess risk for CVD.⁸

Metabolic syndrome combines the cardiovascular risk factors of visceral adiposity, dyslipidemia, hypertension and insulin resistance. Adipose tissue is an active endocrine and paracrine organ that releases adipokines and bioactive mediators that influence not only body weight homeostasis but also insulin resistance, diabetes, lipid levels, coagulation, fibrinolysis, inflammation and atherosclerosis.⁹ Dyslipidemia in obesity is characterized by a spectrum of qualitative lipid abnormalities which include elevated triglycerides (TGs), non-esterified fatty acids (NEFAs), increased levels of low density lipoprotein (LDL) and low levels of high density lipoprotein (HDL) cholesterol levels. Increased plasma NEFA and TGs are associated with insulin resistance¹⁰, type II diabetes mellitus (T2DM)" and increased risk of CVD^{2,13}. High levels of LDL cholesterol and low levels of HDL cholesterol are also emerging cardiovascular risk factors.¹⁴

Insulin resistance is a pathophysiological condition in which a normal insulin concentration does not adequately produce a normal insulin response in the peripheral target tissues such as adipose, muscle, and liver. Under this condition, pancreatic beta cell secretes more insulin (i.e. hyperinsulinemia) to overcome the hyperglycemia among insulin-resistant individuals. Insulin resistance and hyperinsulinemia lead to sympathetic nervous system activation, increased sodium reabsorption, elevated cardiac output, and vasoconstriction. These combine to cause hypertension which contributes to a cycle of worsening cardiovascular risk due to its association with obesity, glucose intolerance, and dyslipidemia. In cardiovascular tissue, insulin resistance leads to overstimulation of the insulin-like growth factor (IGF) pathway, which directly leads to myocardial hypertrophy¹⁵. IGF is a central metabolism regulating hormone, capable of stimulating glucose uptake, glycogen synthesis, lipogenesis, and prevention of proteolysis, similarly to insulin. Insulin like growth factor binding protein 2 (IGFBP2), a 36kDa plasma protein mainly produced by liver and heart¹⁶, has been proposed to bind and inhibit IGF-1 and -2.^{17,18} IGFBP2 is mainly produced by the liver and the heart¹⁶, localizes preferentially in cardiac muscle in isolated perfused heart.^{18,19} Evidence suggests that IGFBP2 contributes to regulation of normal metabolism²⁰ and that is in turn regulated by metabolism and metabolic disorders.²¹ Previous studies have shown that circulating levels of IGFBP2 are reduced in obesity^{17,21} and in diabetics with metabolic syndrome²² but is inversely related to levels of free IGF-1.^{17,18}

Low IGFBP2 concentration is associated with multiple cardiovascular risk factors in patients with type II diabetes.²¹ Conversely, high IGFBP2 levels as seen in transgenic mice expressing human IGFBP2 (hIGFBP2) appears to prevent diet induced obesity and insulin resistance²³ and to lower hypertension associated with obesity and aging²³. Moreover IGFBP2 overexpression demonstrated to correct hyperglycemia and hyperinsulemia in diabetic mice.²⁴ These findings strongly suggest a role for IGFBP2 contribution to maintenance of metabolic function and improvement of cardiometabolic risk factors.

Systemically administered adeno-associated virus 9 (AAV-9) mediated gene transfer has been shown to transduce in cardiomyocytes²⁵ and may represent an attractive approach for cardiovascular disease therapy²⁶.

### SUMMARY OF THE INVENTION:

The present invention relates to methods and pharmaceutical compositions for the treatment of left ventricular hypertrophy and dysfunction associated to metabolic syndrome. In particular, the present invention is defined by the claims.

### DETAILED DESCRIPTION OF THE INVENTION:

Metabolic syndrome (MetS) is a known risk factor for cardiovascular events and is characterized by central obesity plus any two of dyslipidemia, insulin resistance, and hypertension. Obese and diabetic patients with MetS have low plasma insulin-like growth factor binding protein 2 (IGFBP2). The aim of the inventors was to investigate IGFBP2 in the plasma and cardiac tissue of MetS patients. The also investigated IGFBP2 left ventricular expression in mice with MetS and investigated whether adeno-associated virus 9 carrying human IGFBP-2 (AAV9-hIGFBP2) gene therapy could reduce the left ventricular hypertrophy associated to MetS. They found that IGFBP2 heart expression as well as plasmatic levels were decreased in patients with MetS compared to control patients. In a mouse model of diet-induced MetS, cardiac IGFBP2 expression was also decreased and associated with left ventricular hypertrophy. They show for the first time that cardiac overexpression of human IGFBP2 by a single AAV9-hIGFBP2 injection restored cardiac IGFBP2 levels in mouse heart and prevented left ventricle wall thickening, hypertrophy and dysfunction. Restoration of cardiac IGFBP-2 expression level prevents left ventricular hypertrophy and dysfunction associated to MetS. The inventors thus propose that IGFBP2 is a new cardiac therapeutic target in MetS.

Accordingly the first object of the present invention relates to a method of treating left ventricular hypertrophy and dysfunction associated to metabolic syndrome in a subject in need thereof comprising administering to the subject a therapeutically effective amount of at least one agent capable of raising the expression of IGFBP2.

As used herein, the term "Metabolic Syndrome" is present if a person has three or more of the following symptoms: abdominal obesity, hyperglyceridemia, low HDL cholesterol, high blood pressure, and high fasting plasma glucose. The criteria for these symptoms are defined in the third Report of the National Cholesterol Education Program Expert Panel in Detection, Evaluation and Treatment of High blood Cholesterol in Adults (Ford, ES. et al. 2002).

In some embodiments, the subject is obese. The term "obesity" refers to a condition characterized by an excess of body fat. The operational definition of obesity is based on the Body Mass Index (BMI), which is calculated as body weight per height in meter squared (kg/m²). Obesity refers to a condition whereby an otherwise healthy subject has a BMI greater than or equal to 30 kg/m², or a condition whereby a subject with at least one co-morbidity has a BMI greater than or equal to 27 kg/m². An "obese subject" is an otherwise healthy subject with a BMI greater than or equal to 30 kg/m² or a subject with at least one co-morbidity with a BMI greater than or equal 27 kg/m². A "subject at risk of obesity" is an otherwise healthy subject with a BMI of 25 kg/m² to less than 30 kg/m² or a subject with at least one co-morbidity with a BMI of 25 kg/m² to less than 27 kg/m². The increased risks associated with obesity may occur at a lower BMI in people of Asian descent. In Asian and Asian-Pacific countries, including Japan, "obesity" refers to a condition whereby a subject with at least one obesity-induced or obesity-related co-morbidity that requires weight reduction or that would be improved by weight reduction, has a BMI greater than or equal to 25 kg/m². An "obese subject" in these countries refers to a subject with at least one obesity-induced or obesity-related co-morbidity that requires weight reduction or that would be improved by weight reduction, with a BMI greater than or equal to 25 kg/m². In these countries, a "subject at risk of obesity" is a person with a BMI of greater than 23 kg/m2 to less than 25 kg/m².

The term "treatment" means any treatment of a disease in a patient including: (i) preventing the disease, that is causing the clinical symptoms not to develop; (ii) inhibiting the disease, that is, arresting the development of clinical symptoms; and/or (iii) relieving the disease, that is, causing the regression of clinical symptoms. By way of example only, treating may include, preventing or reducing cardiac hypertrophy and/or alleviating symptoms, including, but not limited to exertional dyspnea, fatigue, chest pain, and combinations thereof. As used herein, the term "ameliorate", when used in reference to the severity of a pathologic condition, means that signs or symptoms associated with the condition are lessened, or improvement in the subject's condition. The signs or symptoms to be monitored will be characteristic of a particular pathologic condition and will be well known to skilled clinician, as will the methods for monitoring the signs and conditions. In some embodiments, the method of the invention may reduce death or hospitalization. In particular the method of the invention is suitable for reducing the risk or progression of heart failure, increasing the left ventricle ejection fraction (LVEF), inhibiting left ventricle enlargement, reducing left ventricle end systolic volume, reducing left ventricle end diastolic volume, ameliorating left ventricle dysfunction, and improving myocardial contractibility.

As used herein, the term "IGFBP2" for "Insulin-like Growth Factor-Binding Protein 2" denotes a protein which serves as a carrier protein for Insulin-like growth factor 1 (IGF I) or Insulin-like growth factor 2 (IGF II). An exemplary human amino acid sequence is represented by SEQ ID NO:1 and an exemplary nucleic acid sequence is represented by SEQ ID NO:2.

As used herein the term "agent that raises the expression of IGFBP2" refers to any compound that is able to raise the expression of IGFBP2 in cardiomyocytes. Typically, the agent is selected from the group consisting of polypeptides, nucleic acids, and small organic molecules.

In some embodiments, the agent is an IGFBP2 polypeptide. In some embodiments, the agent is a polypeptide having at least one 70% of identity with SEQ ID NO: 1.

According to the invention a first amino acid sequence having at least 70% of identity with a second amino acid sequence means that the first sequence has 70; 71; 72; 73; 74; 75; 76; 77; 78; 79; 80; 81; 82; 83; 84; 85; 86; 87; 88; 89; 90; 91; 92; 93; 94; 95; 96; 97; 98; 99 or 100% of identity with the second amino acid sequence. Amino acid sequence identity is typically determined using a suitable sequence alignment algorithm and default parameters, such as BLAST P (Karlin and Altschul, 1990). In particular the polypeptide of the invention is a functional conservative variant of IGFBP2. As used herein the term "function-conservative variant" are those in which a given amino acid residue in a protein or enzyme has been changed without altering the overall conformation and function of the polypeptide, including, but not limited to, replacement of an amino acid with one having similar properties (such as, for example, polarity, hydrogen bonding potential, acidic, basic, hydrophobic, aromatic, and the like). Accordingly, a "function-conservative variant" also includes a polypeptide which has at least 70 % amino acid identity and which has the same or substantially similar properties or functions as the native or parent protein to which it is compared. Functional properties of the polypeptide of the invention could typically be assessed in any functional assay as described in the EXAMPLE.

In some embodiments, the polypeptide is fused to a cell-penetrating peptide, a Transactivator of Transcription (TAT) cell penetrating sequence, a cell permeable peptide or a membranous penetrating sequence. The term "cell-penetrating peptides" are well known in the art and refers to cell permeable sequence or membranous penetrating sequence such as penetratin, TAT mitochondrial penetrating sequence and compounds (Bechara and Sagan, 2013; Jones and Sayers, 2012; Khafagy el and Morishita, 2012; Malhi and Murthy, 2012). In a particular embodiment, the heterologous polypeptide is an internalization sequence derived either from the homeodomain of Drosophila Antennapedia/Penetratin (Antp) protein (amino acids 43-58) or the Transactivator of Transcription (TAT) cell penetrating sequences.

In some embodiments, the agent is a nucleic acid encoding for an IGFBP2 polypeptide as above described. In some embodiments, the agent is a nucleic acid encoding for a polypeptide having at least 70% of identity with SEQ ID NO:1. In some embodiments, the agent is a nucleic acid having at least 70% of identity with SEQ ID NO:2.

Typically the nucleic acid as above described is delivered in vivo alone or in association with a vector. In its broadest sense, a "vector" is any vehicle capable of facilitating the transfer of the nucleic acid to the cells and in particular cardiomyocytes. In particular, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rous sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Typically viral vectors are based on non-cytopathic eukaryotic viruses in which non-essential genes have been replaced with the gene of interest. Non-cytopathic viruses include retroviruses (e.g., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Kriegler, 1990 and in Murry, 1991).

Preferred viruses are the adenoviruses and adeno-associated (AAV) viruses, which are double-stranded DNA viruses that have already been approved for human use in gene therapy. Actually 12 different AAV serotypes (AAV1 to 12) are known, each with different tissue tropisms (Wu, Z Mol Ther 2006; 14:316-27). The adeno-associated virus type 1 to 12 can be engineered to be replication deficient and is capable of infecting a wide range of cell types and species (Wu, Z Mol Ther 2006; 14:316-27). It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion. In some embodiments, the nucleic acid as above described is packaged in a cardiotropic adeno-associated viral vector, such AAV9.

Other vectors include plasmid vectors. Plasmid vectors have been extensively described in the art and are well known to those of skill in the art. See e.g. Sambrook et al., 1989. In the last few years, plasmid vectors have been used as DNA vaccines for delivering antigen-encoding genes to cells in vivo. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUC19, pRC/CMV, SV40, and pBlueScript. Other plasmids are well known to those of ordinary skill in the art. Additionally, plasmids may be custom designed using restriction enzymes and ligation reactions to remove and add specific fragments of DNA. Plasmids may be delivered by a variety of parenteral, mucosal and topical routes. For example, the DNA plasmid can be injected by intramuscular, intradermal, subcutaneous, or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate and microencapsulation.

Typically, the nucleic acid sequence is under the control of a heterologous regulatory region, e.g., a heterologous promoter that will drive the expression of the nucleic acid. In some embodiments, the nucleic acid sequence is under the control of a heterologous promoter that will drive the expression of the nucleic acid specifically in cardiomyocyte (e.g. NFAT promoter).

Typically, the agent is administered to the subject in a therapeutically effective amount. By a "therapeutically effective amount" is meant a sufficient amount of the agent of the present invention for reaching a therapeutic effect (e.g. treating cancer). It will be understood, however, that the total daily usage of the compounds and compositions of the present invention will be decided by the attending physician within the scope of sound medical judgment. The specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific compound employed; the duration of the treatment; drugs used in combination or coincidential with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Typically, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, typically from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

Typically the agent of the present invention is combined with pharmaceutically acceptable excipients, and optionally sustained-release matrices, such as biodegradable polymers, to form therapeutic compositions. "Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. Typically, the pharmaceutical compositions contain vehicles, which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The polypeptide or fusion protein of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active compounds in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. The preparation of more, or highly concentrated solutions for direct injection is also contemplated, where the use of DMSO as solvent is envisioned to result in extremely rapid penetration, delivering high concentrations of the active agents to a small tumor area. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. Some variation in dosage will necessarily occur depending on the condition of the subject being treated. The person responsible for administration will, in any event, determine the appropriate dose for the individual subject.

The invention will be further illustrated by the following figures and examples. However, these examples and figures should not be interpreted in any way as limiting the scope of the present invention.

### FIGURES:

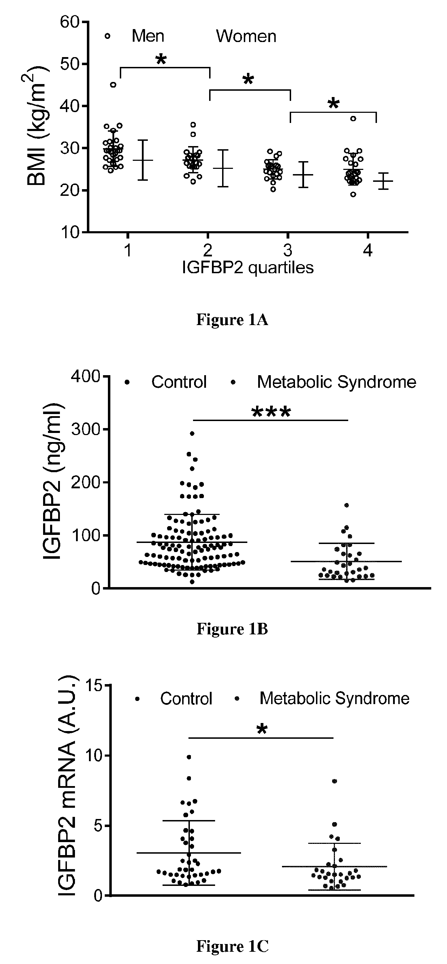

**Figure 1**. (A) Plasma IGFBP2 levels, expressed in quartiles, in relation to BMI in men and women. (B) Plasma IGFBP2 levels in MetS and control patients. (C) IGFBP2 expression levels in heart samples from MetS and control patients.

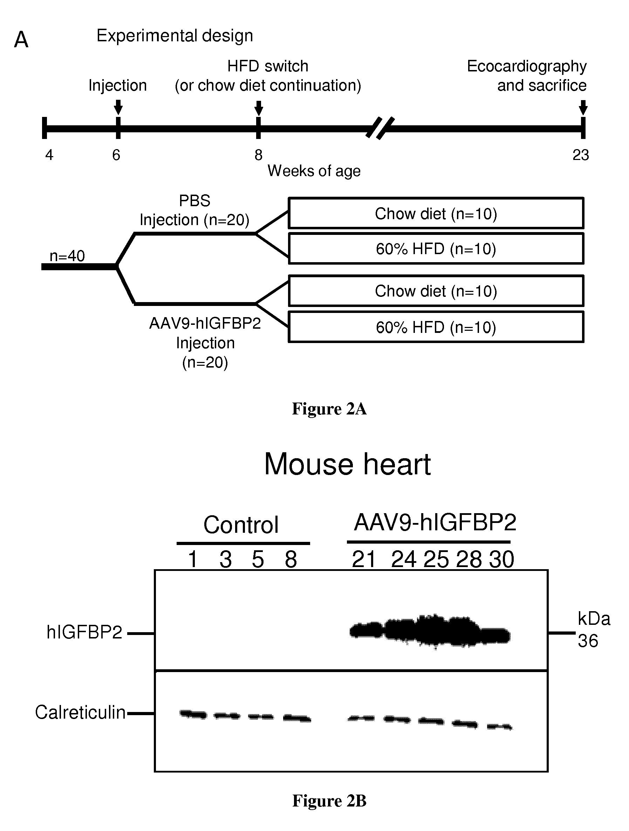

**Figure 2**. (A). Experimental design. Mice were injected with AAV9-hIGFBP2 or PBS at 6 weeks of age. Two weeks after the injection, The mice where switched to 60% HFD or continued to receive chow diet for 14 weeks. (B) Specific western blot detection of human IGFBP2 in left ventricular protein extract from control and AAV9-hIGFBP2 mice. Anti-Calreticulin was used as a control for loading and transfer.

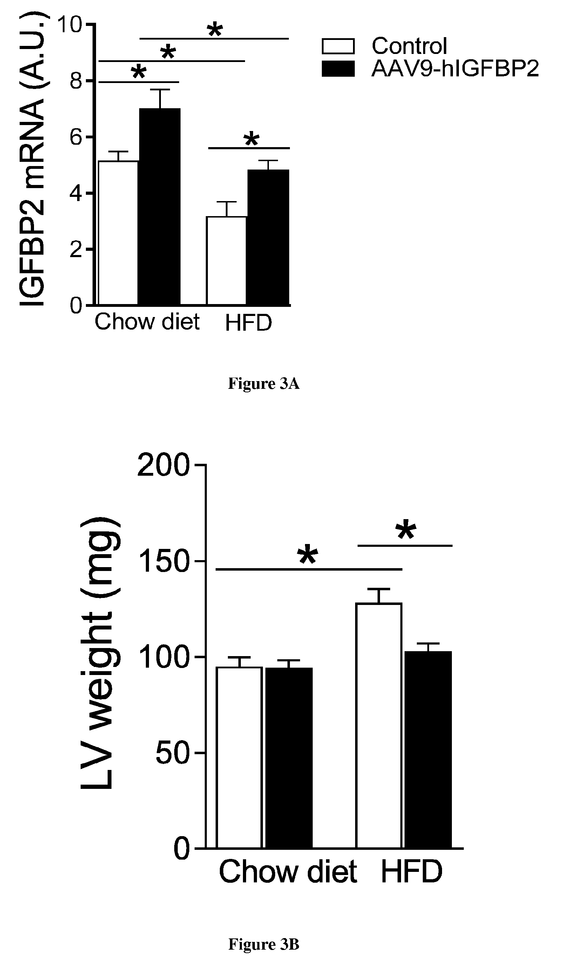

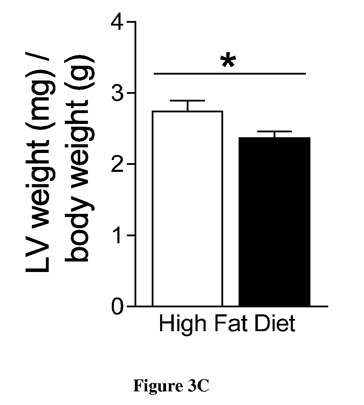

**Figure 3**. (A) Expression of IGFBP2 in control (white bar) and AAV9-hIGFBP2 (black bar) mice fed with chow diet or high fat diet (HFD) as indicated in the materials and methods. (B) Left ventricular (LV) weight (mg) and (C) left ventricular weight (mg) / body weight (g) ratio in mice fed with HFD. *p<0.05.

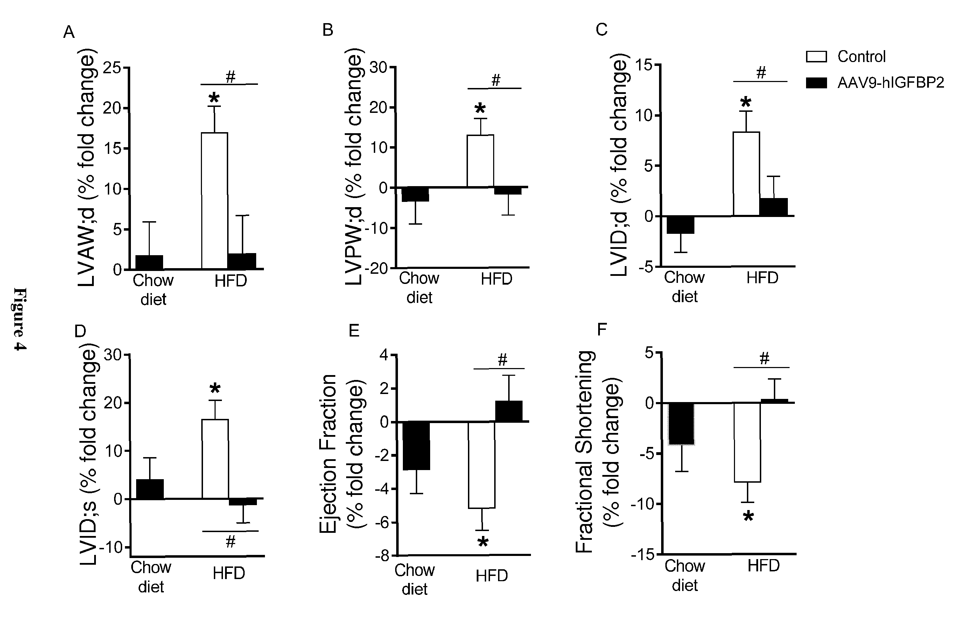

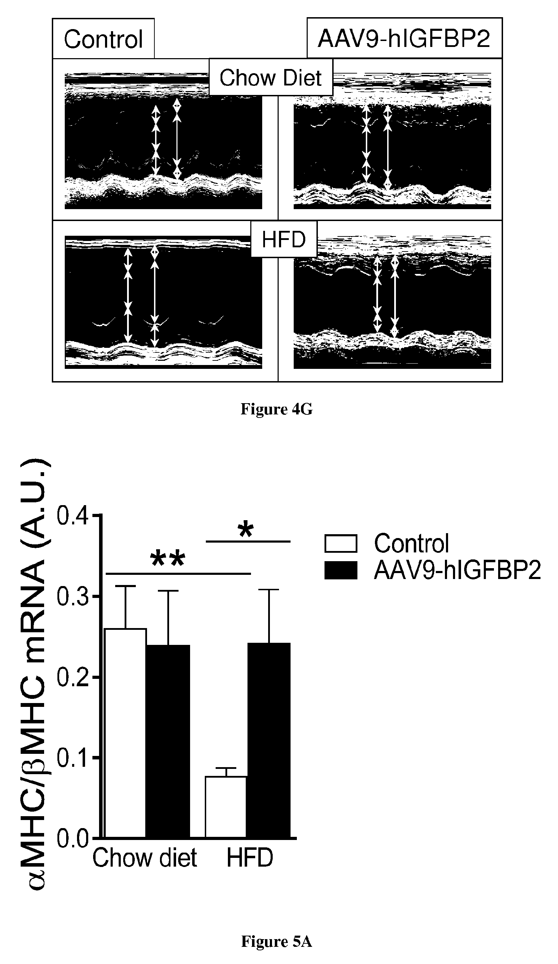

**Figure 4****.** Echocardiographic parameters of mice injected with PBS (white bar) or with AAV9-hIGFBP2 (black bar) and fed with chow diet or HFD. (A). IVS; d represents inter ventricular septum during diastole. (B) LVPW;d is the left ventricular posterior wall during diastole. (C) LVID;d is the left ventricular internal diameter in diastole. (D) LVID;s is the left ventricular internal diameter in systole. (E) Fractional shortening percentage (FS, %). (F) Ejection fraction percentage (EF, %). (G) Representative echocardiographic images in movement mode from parasternal short axis view of hIGFBP2 and control mice fed with chow diet or HFD, *p<0.05 **p<0.01.

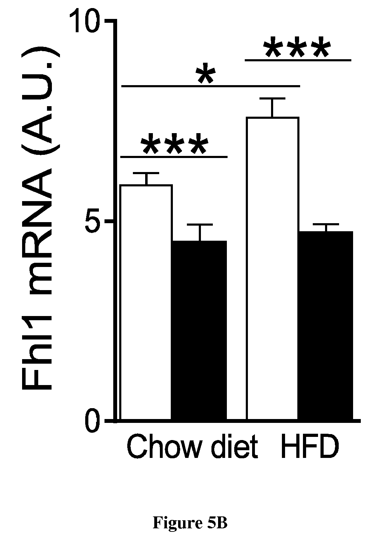

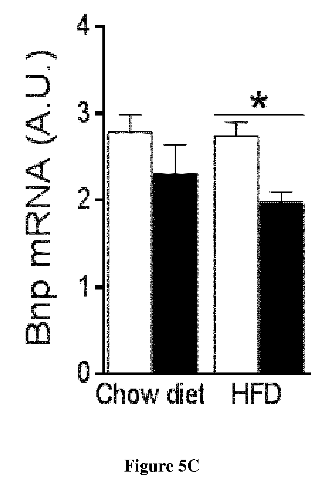

**Figure 5****.** Molecular analysis of heart hypertrophy gene expression in control and AAV9- hIGFBP2 injected mice. (A) αMHC (alpha-myosin heavy chain)/ βMHC (beta-myosin heavy chain). (B) Fhll (Four-and-a-half-LIM domains 1) and (C) BNP (brain natriuretic peptide) expression levels in the LV.

### EXAMPLE:

### Material & Methods

### Study populations

The study population consisted of two independent cohorts. The first cohort was recruited for the measurement of IGFBP-2 plasmatic levels and included 145 patients from CDPA (Centre de Dépistage et de Prevention de l'Athérosclérose / Center for screening and prevention of Atherosclerosis) at the Toulouse Hospital Cardiology Department. Briefly, patients underwent to a clinical examination, echocardiographic analysis and blood pressure measurement. The waist circumference was measured at the high point of the iliac crest at minimal respiration to the nearest 0.1cm. Blood was collected from fasted patients and plasma was obtained after centrifugation and stored at -80°C. Glucose, triglycerides, HDL, LDL and cholesterol concentrations were measured following standard procedures.

### Human cardiac IGFBP2 expression:

A second cohort of patients who underwent to cardiac surgery (aortocoronary bypass, aortic valve replacement, valve replacement, *carotid endarterectomy*) was considered. Samples from right auricle appendages were collected from the Department of Cardiovascular Surgery of Toulouse University Hospital at the beginning of cardiac surgery. Samples were immediately washed in cold buffer, snap-frozen in liquid nitrogen and maintained at -80°C until analysis. All patients underwent to a clinical examination and a left ventricular ejection fraction measurement. According to the IDF definition, for our study we considered that patients had metabolic syndrome if they fulfill the following criteria: central adiposity (waist circumference> 80 cm for women and 94 cm for men) plus at least two risk factors among increased fasting plasma glucose (≥100 mg/dl) or diabetes, reduced high-density lipoproteins (HDL) (<40 mg/dl for men and 50 mg/dl for women or dyslipidemia, elevated plasma triglyceride (TG) levels (≥150 mg/dl) or dyslipidemia and hypertension.

The protocol was approved by the institution's human research (COSSEC), the Commission nationale de l'informatique et des libertés (CNIL) and regional ethics committee (Comité De Protection des Personnes (CPP) # DC 2008-452). Written informed consent was obtained from all participants.

### Circulating IGFBP2 analysis

Circulating levels of human IGFBP2 were measured as previously described.²⁷

### Statistical analysis

Single comparison was performed by 2-tailed unpaired Student's *t* test or Mann-Whitney U test when normality was not met. Multiple comparisons were analyzed by one-way Anova, following, when appropriate, by Dunnet post-hoc test. Graph Pad Prism software 6.0 (www.graphpad.com) was used for all calculations. A P value of 0.05 or less was considered significant. Data are expressed as mean ± SEM (or SD, when specified).

### Results

### Cardiac IGFBP-2 expression and plasmatic IGFBP-2 levels are decreased in metabolic syndrome patients.

The clinical and echocardiographic parameters of the patients tested for IGFBP2 plasmatic levels were recorded (Table I). Plasmatic levels of IGFBP2 shown in quartiles were inversely related to BMI both for man and woman (Figure 1-A). Circulating levels of IGFBP2 were significantly decreased in patients with metabolic syndrome (Figure 1-B). IGFBP2 quantitative expression in heart biopsies from patients with metabolic syndrome (Table II) revealed a significant decreased expression of IGFBP2 (Figure 1-C).

### AAV9-human IGFBP2 transduction and overexpression in the mouse heart.

Mice at 6 weeks of age were injected with AAV9-hIGFBP2 (n=20) or phosphate buffer saline (Control, PBS) (n =20) in the left jugular vein (**Figure 2-A** see also methods). Using an IGFBP2 antibody specific to the human protein, we could detect a strong signal of this protein in the mouse LV, 16 weeks after a single injection of the AAV9-hIGFBP2 in the left jugular vein (**Figure 2-B**).

### High fat diet reduces the expression of IGFBP2 in the left ventricle.

Two weeks after the injection of the AAV9-hIGFBP2 or PBS, the mice were switched to a 60% high-fat diet for 14 weeks or continued to be given the chow diet (see experimental design in **Figure 2-A**)**.** We show that mice injected with either PBS or AAV9-hIGFBP2 and fed with HFD for 14 weeks showed a decrease (50 % and 30 % respectively) in the expression of IGFBP-2 in the left ventricle when compared to lean mice that received normal diet for 14 weeks (**Figure 3**-**A**). However, we observed the IGFBP2 expression level in heart from mice injected with AAV9-hIGFBP2 was higher than in hearts from control either in chow diet or HFD (**Figure 3-A).**

### AAV9-hIGFBP-2 protects left ventricular geometry and function in HFD fed mice.

Left ventricular weight was increased by 40 % in control mice (but not in AAV9-hIGFBP2 injected mice) fed with HFD for 14 weeks in comparison to mice fed with chow diet (**Figure 3-B**). AAV9-hIGFBP2 mice fed with HFD showed a significant reduced LV weight and ratio LV weight/body weight in comparison to control mice fed with HFD (**Figures 3-B** **and** **C**).

Echocardiography parameters (**Figure 4**) analysis of control mice showed a significant increase in the inter-ventricular septum during diastole **(IVS;d)**(**A**) and left ventricular posterior wall during diastole (LVPW;d) (**B**), as well as in the left ventricular diameter both in diastole (LVID;d) (**C**) and systole (LVID;s) (**D**). Fractional Shortening (FS %) (**E**) and Ejection Fraction (EF %) (**F**) were significantly decreased. Interestingly, AAV9-hIGFBP2 mice fed with HFD do not show any enlargement in IVS;d, LVPW;d, LVID;d, LVID;s, (**Figure 4A**-**F**) nor a decrease in FS % and EF% when compared to PBS mice fed with HFD for 14 weeks. No differences were observed when PBS and AAV9-hIGFBP2 mice fed with chow diet were compared.

Accordingly, the expression ratio αMHC/βMHC was strongly decreased when PBS mice were HFD fed, but not in AAV9-hIGFBP2 mice (**Figure 5**-**A**). In addition, the Four And A Half LIM Domains 1 (FHL1) expression was increased in PBS mice fed with HFD. Both groups of AAV9-hIGFBP2 mice (fed with chow diet or HFD) displayed a reduced expression of *fhl1* when compared to respective counterpart injected with PBS (**Figure 5-B**). Brain natriuretic peptide (BNP) gene expression did not shown any differences between PBS mice fed with HFD and chow diet, but show a significant decrease in heart from AAV9-hIGFBP2 mice fed with HFD when compared to PBS mice fed with HFD (**Figure 5-C**).

### AAV9-hIGFBP-2 effect on plasma biochemical parameters.

Plasma biochemical parameters showed an increase for glucose, total cholesterol, HDL and LDL cholesterol when PBS mice where fed with 60% HFD for 14 weeks. AAV9-hIGFBP2 reduced body weight and plasma glucose concentration in mice fed with HFD, but not HDL, LDL nor total cholesterol levels (**Table III**). Non esterified fatty acids (NEFA) were increased in HFD mice injected with AAV9-hIGFBP2.

### Discussion:

We show here decreased IGFBP2 plasmatic levels in relation to increased BMI in both man and woman and report reduced IGFBP2 plasmatic levels in patients with metabolic syndrome. These results are in line with previous studies demonstrating low plasmatic IGFBP2 levels in obese patients^{17,21} and are independently associated with an increased risk of metabolic syndrome in diabetic patients²². Moreover low circulating IGFBP2 is associated with multiple cardiovascular risk factors. Obesity and metabolic syndrome are closely related to CVD^{1,2}. In obesity and Type 2 diabetes, insulin resistance leads to chronic hyperinsulinemia. Increased insulin levels lead to reduced levels of IGFBP2 in the blood and were supposed to reduce IGFBP2 synthesis locally in other tissues.³¹

For the first time, we prove that IGFBP2 expression level is decreased in heart samples from patients with MetS compared to patients without MetS. This is supported by our mouse model of diet-induced metabolic syndrome, were endogenous IGFBP2 expression is decreased in the left ventricle compared to control mice.

Our mice model of metabolic syndrome fed with HFD for 14 weeks display increased LV weight, LV posterior wall, LV inter-ventricular septum during diastole and LV diameter in systole and diastole. Left ventricular ejection fraction and fractional shortening were also impaired according to an eccentric pattern of LV hypertrophy, abundantly observed and reviewed in MetS and obesity in human^{32,33} and animal models.³⁴

We then investigated whether adeno-associated virus 9 carrying human IGFBP-2 (AAV9-hIGFBP2) gene therapy could improve the MetS-associated left ventricular dysfunction. In this study, we over-expressed hIGFBP2 in mice heart by a single AAV9 injection. IGFBP2 closely regulates IGF-1 trough an inhibitory effect. IGF-1, increased in the presence of hyperinsulinemia, is responsible of cell growth and proliferation³⁵, increases the protein synthesis³⁶ and the size of cardiomyocytes³⁷ in vitro and induces cardiac hypertrophy in vivo³⁸. Interestingly, heart cells derived from the lizard Iguana iguana has demonstrated to secrete IGFBP in the medium, to localize to the cells membranes and to bind/inhibits IGF-I in a specific and high-affinity manner³⁹, leading to the hypothesis that IGFBP has a paracrine effect. We postulated that increasing IGFBP2 levels in left ventricle could reduce left ventricular hypertrophy in HFD-induced metabolic syndrome by sequestering IGF-1.

Effectively, mice injected with AAV9-hIGFBP2, overexpressing human IGFBP2 in LV and fed with HFD were protected from HFD-induced LV hypertrophy, with prevention in LV mass, LV weight/body weight, interventricular septum, posterior wall and internal diameter increase (compared to HFD fed control mice). The expression of hIGFBP2 via AAV9 was also able to prevent the ejection fraction and fractional shortening decrease. The protection against LV hypertrophy was also demonstrated at the molecular level. We show this by assessing the αMHC/βMHC expression ratio and FHL1 expression level which were respectively decreased⁴¹ and increased⁴¹ in heart of human and mice exhibiting hypertrophic cardiomyopathy. We found a significant increase in the αMHC/βMHC expression ratio and a decreased expression of FHL1 in HFD-fed mice expressing hIGFBP2. Moreover, the expression of brain natriuretic peptide (BNP) was significantly reduced in HFD mice injected with hIGFBP2 compared to control mice. We propose that the reestablishment of IGFBP2 level in heart could improve left ventricular hypertrophy consequent to obesity and metabolic syndrome.

A previous study of IGFBP2- transgenic mice demonstrated that IGFBP-2 overexpression prevents the development of obesity, its metabolic and systolic blood pressure measured by tail-cuff plethysmography²³. Moreover, IGFBP2 overexpression in insulin-resistant ob/ob by adenovirus injection reversed diabetes demonstrating that IGFBP2 can regulate glucose metabolism, a finding with potential implications for the pathogenesis and treatment of diabetes²⁴. In our mouse model of metabolic syndrome, C57BL/6J mice fed with HFD for 14 weeks display obesity, increased glucose levels, triglycerides and LDL-cholesterol. The overexpression of IGFBP2 in heart during the chow diet showed no abnormal characteristics in plasma biochemical parameters, in LV weight or echocardiographic parameters. The AAV9-hIGFBP2 HFD-fed mice displayed amelioration for plasma glucose levels but no improvement in plasmatic HDL, LDL or total cholesterol levels and increased NEFA circulating levels, indicating that the protective effect of human IGFBP2 against LV hypertrophy induced by metabolic syndrome is more likely due to the IGFBP2 produced and secreted by the cardiomyocytes than to IGFBP2 systemic action in lightening metabolic syndrome components.

Cardiac AAV9 gene therapy has demonstrate its long-term therapeutic effectiveness and safety in preclinical small⁴² and large animal models^{26,43}. There are multiple human AAV gene therapy trials^{43,44}, however very recent studies report genotoxicity⁴⁵ and proto-oncogene activation⁴⁶ in liver. Careful design of enhancer and promoter elements may minimize the risks of insertional mutagenesis and may let to the fulfillment of the promising effect of AAV gene therapy in multiple diseases.

**Table I. Clinical characteristic of cohort for IGFBP2 plasmatic levels**

| Parameter | Control | Metabolic Syndrome | *P* Value |
|---|---|---|---|
| N | 115 | 30 | |
| Age | 58±11 | 55±13 | 0.2027 |
| Woman %, (n) | 40.86(47) | 30(9) | 0.2794 |
| BMI, kg/m² TOT | 24.8±3.33 | 30.08±4.45 | <0.0001 |
| Waist circumference (cm) | 88.19 ± 10.5 | 101.89 ± 13.05 | <0.0001 |
| Type II Diabetes %, (n) | 2.6 (3) | 23.33(7) | <0.0001 |
| TG g/L | 1.11 ± 0.62 | 2.11 ± 1.13 | <0.0001 |
| Cholesterol g/L | 2.07 ± 0.43 | 1.98 ± 0.47 | 0.3183 |
| LDL, g/L | 1.23 ± 0.4 | 1.103 ± 0.36 | 0.1164 |
| HDL, g/L | 0.62 ± 0.2 | 0.44 ± 0.14 | <0.0001 |
| Dyslipidemia, % (n) | 64.34 (74) | 80 (24) | 0.1230 |
| Current smokers, % (n) | 17.39 (20) | 20(6) | 0.7422 |
| SBP, mmHg | 129.41 ± 15.8 | 135.3 ± 11.64 | 0.0583 |
| DBP, mmHg | 80.81 ± 11.08 | 84.6 ± 8.24 | 0.0823 |
| Heart rate, bpm | 63.41 ± 9.89 | 68.69 ± 13.09 | 0.0165 |
| LVM indexed | 72.89 ± 21.48 | 76.63 ± 15.46 | 0.3728 |
| LVEF, % | 66.28 ± 7.96 | 60.71 ± 7.09 | 0.0006 |
| NYHA 0/I/II/III/IV | 1/95/14/4/1 | 0/17/7/3/0 | 0.0495 |
| E/A ratio | 0.97 ± 0.32 | 1.03 ± 0.33 | 0.365 |

| | | | |
|---|---|---|---|
| Average ± SD, BMI, body mass index, TG, triglycerides, LDL, low density lipoproteins, HDL, high density lipoproteins, SBP, systolic blood pressure, DBP diastolic blood pressure, LVM, left ventricular mass, LVEF, left ventricular ejection fraction, NYHA, New York Heart Association classification, E/A, ratio of the early (E) to late (A)ventricular filling velocities | | | |

**Table II Clinical characteristic of cohort for mRNA IGFBP2 expression in heart samples**

| Parameter | Control | Metabolic Syndrome | *P* Value |
|---|---|---|---|
| N | 38 | 27 | |
| Age | 65 ± 13 | 67 ± 10 | 0.5287 |
| Woman % (n) | 15.79 (6) | 21.43 (6) | 0.5642 |
| BMI, kg/m² | 25.19 ± 3.21 | 30.39 ± 4.08 | <0.0001 |
| Waist circumference (cm) | 88.79 ± 8.22 | 102.5 ± 11.32 | <0.0001 |
| Type II Diabetes, % (n) | 10.52 (4) | 70.37 (19) | <0.0001 |
| Dyslipidemia, % (n) | 68.42 (26) | 62.96 (17) | <0.0001 |
| Current Smokers, % (n) | 13.15 (5) | 14.81 (4) | 0.8847 |
| HTN, % (n) | 57.89 (22) | 85.18 (23) | 0.0261 |
| LVEF, % | 57.18 ± 12.06 | 60.59 ± 6.78 | 0.1898 |
| NYHA I/II/III/IV | 6/6/13/0 | 5/11/4/0 | 0.1643 |
| NT-proBNP, (n) | 1028 ± 949,99 (10) | 1507 ± 1993.49 (6) | 0.5224 |

| | | | |
|---|---|---|---|
| Average ± SD, BMI, body mass index, LVEF, left ventricular ejection fraction, NYHA, New York Heart association classification | | | |

**Table III. Mouse plasma physiological and biochemical parameters at the end of the experiment**

| Parameter | Chow diet | | HFD | |
|---|---|---|---|---|
| | Control n=10 | AAV9-hIGFBP2 n=10 | Control n=10 | AAV9-hIGFBP2 n=10 |
| Weight (g) | 32.16 ± 0.98 | 29.87 ± 0.58* | 46.21 ± 1.11*** | 43.12 ± 1.33^{$$$#} |
| Plasma Glucose (mmol/L) | 9.7 ± 0.43 | 10.3 ± 0.37 | 11.95 ± 0.58* | 10.53 ± 0.54^{#} |
| Total Cholesterol (mmol/L) | 1.87 ± 0.05 | 1.96 ± 0.07 | 3.91 ± 0.29*** | 3.85 ± 0.16^{$$$} |
| LDL Cholesterol (mmol/L) | 0.19 ± 0.01 | 0.17 ± 0.01 | 0.35 ± 0.02*** | 0.35 ± 0.02^{$$$} |
| HDL Cholesterol (mmol/L) | 1.05 ± 0.03 | 1.11 ± 0.03 | 1.81 ± 0.11*** | 1.78 ± 0.05^{$$$} |
| Triglycerides (mmol/L) | 0.79 ± 0.04 | 0.95 ± 0.07 | 0.89 ± 0.06 | 0.86 ± 0.07 |
| NEFA (mmol/L) | 0.57 ± 0.04 | 0.65 ± 0.03 | 0.45 ± 0.03 | 0.62 ± 0.05# |

| | | | | |
|---|---|---|---|---|
| Control HFD mice showed increased weight, glucose and total, LDL and HDL Cholesterol compared to control mice fed with chow diet. HFD mice injected with AAV9-hIGFBP2 compared to control HFD mice did not show difference for total, LDL and HDL Gholesterol, nor fortryglycerides levels, but for weight, glucose and NEFA levels. Average ± SEM *p<0.5, ***p<0.001 vs Control chow diet, ^{#}p<0.05, ^{###}p<0.001 vs Control HFD,^{$$$} p<0.001 vs AAV9-hIGFBP2 chow diet, NEFA (non-esterified fatty acids) | | | | |

### SEQUENCES:

SEQ ID NO:1_human amino acid sequence_IGFBP2 (NCBI Reference Sequence: NP_000588.2): 
1 mlprvgcpal plppppllpl lpllllllga sgggggarae vlfrcppctp erlaacgppp
61 vappaavaav aggarmpcae lvrepgcgcc svcarlegea cgvytprcgq glrcyphpgs
121 elplqalvmg egtcekrrda eygaspeqva dngddhsegg lvenhvdstm nmlgggsag
181 rkplksgmke lavfrekvte qhrqmgkggk hhlgleepkk lrpppartpc qqeldqvler
241 istmrlpder gplehlyslh ipncdkhgly nlkqckmsln gqrgecwcvn pntgkliqga
301 ptirgdpech lfyneqqear gvhtqrmq
SEQ ID NO:2_human nucleic acid sequence_IGFBP2 (NCBI Reference Sequence: NM_000597.2):

1 tgcggcggcg agggaggagg aagaagcgga ggaggcggct cccgcgctcg cagggccgtg
61 ccacctgccc gcccgcccgc tcgctcgctc gcccgccgcg ccgcgctgcc gaccgccagc
121 atgctgccga gagtgggctg ccccgcgctg ccgctgccgc cgccgccgct gctgccgctg
181 ctgccgctgc tgctgctgct actgggcgcg agtggcggcg gcggcggggc gcgcgcggag
241 gtgctgttcc gctgcccgcc ctgcacaccc gagcgcctgg ccgcctgcgg gcccccgccg
301 gttgcgccgc ccgccgcggt ggccgcagtg gccggaggcg cccgcatgcc atgcgcggag
361 ctcgtccggg agccgggctg cggctgctgc tcggtgtgcg cccggctgga gggcgaggcg
421 tgcggcgtct acaccccgcg ctgcggccag gggctgcgct gctatcccca cccgggctcc
481 gagctgcccc tgcaggcgct ggtcatgggc gagggcactt gtgagaagcg ccgggacgcc
541 gagtatggcg ccagcccgga gcaggttgca gacaatggcg atgaccactc agaaggaggc
601 ctggtggaga accacgtgga cagcaccatg aacatgttgg gcgggggagg cagtgctggc
661 cggaagcccc tcaagtcggg tatgaaggag ctggccgtgt tccgggagaa ggtcactgag
721 cagcaccggc agatgggcaa gggtggcaag catcaccttg gcctggagga gcccaagaag
781 ctgcgaccac cccctgccag gactccctgc caacaggaac tggaccaggt cctggagcgg
841 atctccacca tgcgccttcc ggatgagcgg ggccctctgg agcacctcta ctccctgcac
901 atccccaact gtgacaagca tggcctgtac aacctcaaac agtgcaagat gtctctgaac
961 gggcagcgtg gggagtgctg gtgtgtgaac cccaacaccg ggaagctgat ccaggagccc
1021 cccaccatcc ggggggaccc cgagtgtcat ctcttctaca atgagcagca ggaggctcgc
1081 ggggtgcaca cccagcggat gcagtagacc gcagccagcc ggtgcctggc gcccctgccc
1141 cccgcccctc tccaaacacc ggcagaaaac ggagagtgct tgggtggtgg gtgctggagg
1201 attttccagt tctgacacac gtatttatat ttggaaagag accagcaccg agctcggcac
1261 ctccccggcc tctctcttcc cagctgcaga tgccacacct gctccttctt gctttccccg
1321 ggggaggaag ggggttgtgg tcggggagct ggggtacagg tttggggagg gggaagagaa
1381 atttttattt ttgaacccct gtgtcccttt tgcataagat taaaggaagg aaaagtaaa

### REFERENCES:

Throughout this application, various references describe the state of the art to which this invention pertains. The disclosures of these references are hereby incorporated by reference into the present disclosure.
1. Mottillo S, Filion KB, Genest J, et al. The Metabolic Syndrome and Cardiovascular Risk. J Am Coll Cardiol. 2010;56(14):1113-1132.
2. Gami AS, Witt BJ, Howard DE, et al. Metabolic Syndrome and Risk of Incident Cardiovascular Events and Death. J Am Coll Cardiol. 2007;49(4):403-414.
3. Mancia G, Bombelli M, Corrao G, et al. Metabolic syndrome in the Pressioni Arteriose Monitorate E Loro Associazioni (PAMELA) study: daily life blood pressure, cardiac damage, and prognosis. Hypertension. 2007;49(1):40-47.
4. Alberti KGMM, Zimmet P, Shaw J. The metabolic syndrome - A new worldwide definition. Lancet. 2005;366(9491):1059-1062.
5. World Health Organization. Definition, diagnosis and classification of diabetes mellitus and its complications. Part 1: Diagnosis and classification of diabetes mellitus. Geneva, World Heal Organ. 1999. http://apps.who.int/iris/bitstream/10665/66040/1/WHO_NCD_NCS_99.2.pdf.
6. Executive Summary of The Third Report of The National Cholesterol Education Program Expert Panel on Detection, Evaluation and Treatment of High Blood Cholesterol in Adults (Adult Treatment Panel III). Jama. 2001;285(19):2486-2497.
7. Ford ES. Prevalence of the Metabolic Syndrome Defined by the International Diabetes Federation Among Adults in the U.S. Diabetes Care. 2005;28(11):2745-2749.
8. Brown TM, Voeks JH, Bittner V, Safford MM. Variations in prevalent cardiovascular disease and future risk by metabolic syndrome classification in the REasons for Geographic and Racial Differences in Stroke (REGARDS) study. Am Heart J. 2010;159(3):385-391.
9. Van Gaal LF, Mertens IL, De Block CE. Mechanisms linking obesity with cardiovascular disease. Nature. 2006;444:875-880.
10. Karpe F, Dickmann JR, Frayn KN. Fatty Acids, Obesity, and Insulin Resistance: Time for a Reevaluation. Diabetes. 2011;60(10):2441-2449.
11. Fontbonne A, Eschwège E, Cambien F, et al. Hypertriglyceridaemia as a risk factor of coronary heart disease mortality in subjects with impaired glucose tolerance or diabetes. Results from the 11-year follow-up of the Paris Prospective Study. Diabetologia. 1989;32(5):300-304.
12. Criqui MH, Heiss G, Cohn R, et al. PLASMA TRIGLYCERIDE LEVEL AND MORTALITY FROM CORONARY ARTERY DISEASE. N Engl J Med. 1993;328(17):1220-1225.
13. Miller M, Stone NJ, Ballantyne C, et al. Triglycerides and cardiovascular disease: A scientific statement from the American Heart Association. Circulation. 2011;123(20):2292-2333.
14. National Cholesterol Education Program (NCEP). Expert Panel on Detection, Evaluation, and Treatment of High Blood Cholesterol in Adults (Adult Treatment Panel III). Third Report of the National Cholesterol Education Program (NCEP) Expert Panel on Detection, Evaluation, and Treatment of High Blood Chol. Circulation. 2002;106:3143-3421.
15. Sidó Z, Jákó P, Kneffel Z, Kispéter Z, Pavlik G. Cardiac hypertrophy and diastolic function in physically well trained and in obese men. Int J Obes. 2003;27(11):1347-1352.
16. Wu C, MacLeod I, Su a. I. BioGPS and MyGene.info: organizing online, gene-centric information. Nucleic Acids Res. 2013;41(D1):D561-D565.
17. Nam S, Lee E, Kim K, et al. Effect of obesity on total and free insulin-like growth factor (IGF)-1, and their relationship to IGF-binding protein (BP)-1, IGFBP-2, IGFBP-3, insulin, and growth hormone. Int J Obes Relat Metab Disord. 1997;21(5):355-359.
18. Firth SM, Baxter RC. Cellular actions of the insulin-like growth factor binding proteins. EndocrRev. 2002;23(6):824-854.
19. Boes M, Booth B, Sandra A, Dake B, Bergold A, Bar S. Insulin-like growth factor binding protein (IGFBP)4 accounts for the connective tissue distribution of endothelial cell IGFBPs perfused through the isolated heart. Endocrinology. 1992;131(1):327-330.
20. Sabin MA, Russo VC, Azar WJ, Yau SW, Kiess W, Werther GA. IGFBP-2 at the interface of growth and metabolism--implications for childhood obesity. Pediatr Endocrinol Rev. 2011;8(4):382-393.
21. Frystyk J, Vestbo E, Fisker S. Circulating Levels of Free Insulin-like Growth Factors in Obese Subjects: the Impact of Type 2 Diabetes. Diabetes Metab Res Rev. 1999;3(September):314-322.
22. Heald AH, Kaushai K, Siddals KW, Rudenski AS, Anderson SG, Gibson JM. Insulin-like growth factor binding protein-2 (IGFBP-2) is a marker for the metabolic syndrome. Exp Clin Endocrinol Diabetes. 2006;114(7):371-376.
23. Wheatcroft S, Kearney M, Shah A. IGF-binding protein-2 protects against the development of obesity and insulin resistance. Diabetes. 2007;56(2):285-294.
24. Hedbacker K, Birsoy K, Wysocki RW, et al. Antidiabetic effects of IGFBP2, a leptin-regulated gene. Cell Metab. 2010;11(1):11-22.
25. Pacak C a., Mah CS, Thattaliyath BD, et al. Recombinant adeno-associated virus serotype 9 leads to preferential cardiac transduction in vivo. Circ Res. 2006;99(4):3-9.
26. Pleger ST, Shan C, Ksienzyk J, et al. Cardiac AAV9-S100A1 gene therapy rescues post-ischemic heart failure in a preclinical large animal model. Sci Transl Med. 2011;3(92):92ra64.
27. Berry M, Galinier M, Delmas C, et al. Proteomics analysis reveals IGFBP2 as a candidate diagnostic biomarker for heart failure. IJC Metab Endocr. 2015;6:5-12.
28. Keeler AM, Conlon T, Walter G, et al. Long-term Correction of Very Long-chain Acyl-CoA Dehydrogenase Deficiency in Mice Using AAV9 Gene Therapy. Mol Ther. 2012;20(6):1131-1138.
29. Gardin JM, Siri FM, Kitsis RN, Edwards JG, Leinwand LA. Echocardiographic Assessment of Left Ventricular Mass and Systolic Function in Mice. Circ Res. 1995;76:907-914.
30. Turkieh A, Caubère C, Barutaut M, et al. Apolipoprotein O is mitochondrial and promotes lipotoxicity in heart. J Clin Invest. 2014;124(5):2277-2286.
31. Calle EE, Kaaks R. Overweight, obesity and cancer: epidemiological evidence and proposed mechanisms. Nat Rev Cancer. 2004;4(8):579-591.
32. Guerra F, Mancinelli L, Angelini L, et al. The association of left ventricular hypertrophy with metabolic syndrome is dependent on body mass index in hypertensive overweight or obese patients. PLoS One. 2011;6(1):2-7.
33. Cuspidi C, Rescaldani M, Sala C, Grassi G. Left-ventricular hypertrophy and obesity: a systematic review and meta-analysis of echocardiographic studies. J Hypertens. 2014;32(1):16-25.
34. Abel ED, Litwin SE, Sweeney G. Cardiac Remodeling in Obesity. Physiol Rev. 2008;88(2):389-419.
35. Brauna S, Bitton-Worms K, le Roith D. The link between the metabolic syndrome and cancer. Int J Biol Sci. 2011;7(7):1003-1015.
36. Ito H, Hiroe M, Hirata Y, et al. Insulin-like growth factor-I induces hypertrophy with increased expression of muscle specific genes in cultured rat cardiomyocytes. Circulation. 1993;87:1715-1721.
37. Donath MY, Zapf J, Eppenberger-Eberhardt M, Froesch ER, Eppenberger HM. Insulin-like growth factor I stimulates myofibril development and decreases smooth muscle alpha-actin of adult cardiomyocytes. Proc Natl Acad Sci USA. 1994;91 (March): 1686-1690.
38. Cittadini A, Stromer H, Katz SE, et al. Differential Cardiac Effects of Growth Hormone and Insulin-like Growth Factorl in the Rat. Circulation. 1996;93(4):800-809.
39. Kelley KM, Schmidt KE, Berg L, et al. BEYOND CARRIER PROTEINS Comparative endocrinology of the insulin-like growth factor-binding protein. J Endocrinol. 2002:3-18.
40. Gupta M. Factors controlling cardiac myosin-isoform shift during hypertrophy and heart failure. J Mol Cell Cardiol. 2007;43(4):388-403.
41. Sheikh F, Raskin A, Chu P-H, et al. An FHL1-containing complex within the cardiomyocyte sarcomere mediates hypertrophic biomechanical stress responses in mice. J Clin Invest. 2008;118(12):3870-3880.
42. Ruozi G, Bortolotti F, Falcione A, et al. AAV-mediated in vivo functional selection of tissue-protective factors against ischaemia. Nat Commun. 2015;6(May):7388.
43. Ginn SL, Alexander JE, Edelstein ML, Abedi MR, Wixon J. Gene therapy clinical trials worlwide to 2012- an update. J Gene Med. 2013;15(6):65-77.
44. Greenberg B, Yaroshinsky A, Zsebo KM, et al. Design of a Phase 2b Trial of Intracoronary Administration of AAV1/SERCA2a in Patients With Advanced Heart Failure. JACC Hear Fail. 2014;2(1):84-92.
45. Chandler RJ, Lafave MC, Varshney GK, et al. Vector design influences hepatic genotoxicity after adeno-associated virus gene therapy. J Clin Invest. 2015;125(2):1-11.
46. Russell DW, Grompe M. Adeno-associated virus finds its disease. Nat Genet. 2015;47(10):1104-1105.

## Claims

1. A method of treating left ventricular hypertrophy and dysfunction associated to metabolic syndrome in a subject in need thereof comprising administering to the subject a therapeutically effective amount of at least one agent capable of raising the expression of IGFBP2.

2. The method of claim 1 wherein the subject is obese.

3. The method of claim 1 wherein the agent is an IGFBP2 polypeptide having at least one 70% of identity with SEQ ID NO: 1.

4. The method of claim 1 wherein the agent is a nucleic acid encoding for a polypeptide having at least 70% of identity with SEQ ID NO: 1.

5. The method of claim 1 wherein the agent is a nucleic acid having at least 70% of identity with SEQ ID NO:2.

6. The method of claim 4 or 5 wherein the nucleic acid is delivered alone or in association with a vector.

7. The method of claim 6 wherein the vector is a viral vector.

8. The method of claim 7 wherein the vector is an AAV vector.

9. The method of claim 8 wherein the vector is an AVV9 vector.


SEQUENCE LISTING

<110>  Inserm

<120>  METHODS AND PHARMACEUTICAL COMPOSITIONS FOR THE TREATMENT OF LEFT
       VENTRICULAR HYPERTROPHY AND DYSFUNCTION ASSOCIATED TO METABOLIC
       SYNDROME

<130>  BIO16040 ROUET /MC

<160>  2

<170>  PatentIn version 3.5

<210>  1
<211>  328
<212>  PRT
<213>  Homo sapiens

<400>  1

Met Leu Pro Arg Val Gly Cys Pro Ala Leu Pro Leu Pro Pro Pro Pro
1               5                   10                  15

Leu Leu Pro Leu Leu Pro Leu Leu Leu Leu Leu Leu Gly Ala Ser Gly
            20                  25                  30

Gly Gly Gly Gly Ala Arg Ala Glu Val Leu Phe Arg Cys Pro Pro Cys
        35                  40                  45

Thr Pro Glu Arg Leu Ala Ala Cys Gly Pro Pro Pro Val Ala Pro Pro
    50                  55                  60

Ala Ala Val Ala Ala Val Ala Gly Gly Ala Arg Met Pro Cys Ala Glu
65                  70                  75                  80

Leu Val Arg Glu Pro Gly Cys Gly Cys Cys Ser Val Cys Ala Arg Leu
                85                  90                  95

Glu Gly Glu Ala Cys Gly Val Tyr Thr Pro Arg Cys Gly Gln Gly Leu
            100                 105                 110

Arg Cys Tyr Pro His Pro Gly Ser Glu Leu Pro Leu Gln Ala Leu Val
        115                 120                 125

Met Gly Glu Gly Thr Cys Glu Lys Arg Arg Asp Ala Glu Tyr Gly Ala
    130                 135                 140

Ser Pro Glu Gln Val Ala Asp Asn Gly Asp Asp His Ser Glu Gly Gly
145                 150                 155                 160

Leu Val Glu Asn His Val Asp Ser Thr Met Asn Met Leu Gly Gly Gly
                165                 170                 175

Gly Ser Ala Gly Arg Lys Pro Leu Lys Ser Gly Met Lys Glu Leu Ala
            180                 185                 190

Val Phe Arg Glu Lys Val Thr Glu Gln His Arg Gln Met Gly Lys Gly
        195                 200                 205

Gly Lys His His Leu Gly Leu Glu Glu Pro Lys Lys Leu Arg Pro Pro
    210                 215                 220

Pro Ala Arg Thr Pro Cys Gln Gln Glu Leu Asp Gln Val Leu Glu Arg
225                 230                 235                 240

Ile Ser Thr Met Arg Leu Pro Asp Glu Arg Gly Pro Leu Glu His Leu
                245                 250                 255

Tyr Ser Leu His Ile Pro Asn Cys Asp Lys His Gly Leu Tyr Asn Leu
            260                 265                 270

Lys Gln Cys Lys Met Ser Leu Asn Gly Gln Arg Gly Glu Cys Trp Cys
        275                 280                 285

Val Asn Pro Asn Thr Gly Lys Leu Ile Gln Gly Ala Pro Thr Ile Arg
    290                 295                 300

Gly Asp Pro Glu Cys His Leu Phe Tyr Asn Glu Gln Gln Glu Ala Arg
305                 310                 315                 320

Gly Val His Thr Gln Arg Met Gln
                325

<210>  2
<211>  1439
<212>  DNA
<213>  Homo sapiens

<400>  2
tgcggcggcg agggaggagg aagaagcgga ggaggcggct cccgcgctcg cagggccgtg        60

ccacctgccc gcccgcccgc tcgctcgctc gcccgccgcg ccgcgctgcc gaccgccagc       120

atgctgccga gagtgggctg ccccgcgctg ccgctgccgc cgccgccgct gctgccgctg       180

ctgccgctgc tgctgctgct actgggcgcg agtggcggcg gcggcggggc gcgcgcggag       240

gtgctgttcc gctgcccgcc ctgcacaccc gagcgcctgg ccgcctgcgg gcccccgccg       300

gttgcgccgc ccgccgcggt ggccgcagtg gccggaggcg cccgcatgcc atgcgcggag       360

ctcgtccggg agccgggctg cggctgctgc tcggtgtgcg cccggctgga gggcgaggcg       420

tgcggcgtct acaccccgcg ctgcggccag gggctgcgct gctatcccca cccgggctcc       480

gagctgcccc tgcaggcgct ggtcatgggc gagggcactt gtgagaagcg ccgggacgcc 540

gagtatggcg ccagcccgga gcaggttgca gacaatggcg atgaccactc agaaggaggc 600

ctggtggaga accacgtgga cagcaccatg aacatgttgg gcgggggagg cagtgctggc 660

cggaagcccc tcaagtcggg tatgaaggag ctggccgtgt tccgggagaa ggtcactgag 720

cagcaccggc agatgggcaa gggtggcaag catcaccttg gcctggagga gcccaagaag 780

ctgcgaccac cccctgccag gactccctgc caacaggaac tggaccaggt cctggagcgg 840

atctccacca tgcgccttcc ggatgagcgg ggccctctgg agcacctcta ctccctgcac 900

atccccaact gtgacaagca tggcctgtac aacctcaaac agtgcaagat gtctctgaac 960

gggcagcgtg gggagtgctg gtgtgtgaac cccaacaccg ggaagctgat ccagggagcc 1020

cccaccatcc gggggacccc cgagtgtcat ctcttctaca atgagcagca ggaggctcgc 1080

ggggtgcaca cccagcggat gcagtagacc gcagccagcc ggtgcctggc gcccctgccc 1140

cccgcccctc tccaaacacc ggcagaaaac ggagagtgct tgggtggtgg gtgctggagg 1200

attttccagt tctgacacac gtatttatat ttggaaagag accagcaccg agctcggcac 1260

ctccccggcc tctctcttcc cagctgcaga tgccacacct gctccttctt gctttccccg 1320

ggggaggaag gggttgtggt tcggggagct ggggtacagg tttggggagg gggaagagaa 1380

atttttattt ttgaacccct gtgtcccttt tgcataagat taaaggaagg aaaagtaaa 1439